# EUROPEAN PATENT APPLICATION

(11) **EP 1 829 610 A1**
(43) Date of publication of application: **05.09.2007**
(21) Application number: 05811579.1
(22) Date of filing: 02.12.2005
(51) Int. Cl.: B01J 31/28, C07C 5/09, C07C 11/107, C07C 15/52, C07C 29/17, C07C 33/025, C07C 33/035, C07C 33/22, C07C 35/38, C07C 39/23, C07C 45/62, C07C 49/217, C07C 49/727, C07C 51/36, C07C 57/44, C07C 67/303, C07C 69/618, C07C 209/70, C07C 211/45, C07F 7/08, C07B 61/00

(54) **NOVEL CATALYST**

(30) Priority: 02.12.2004 JP 2004350459
(71) Applicant: Wako Pure Chemical Industries, Ltd., Osaka-shi, Osaka 540-8605 (JP)
(72) Inventor: HIROTA, Kosaku, Gifu-shi, Gifu 5020003 (JP); SAJIKI, Hironao, Gifu-shi, Gifu 5020823 (JP)
(74) Representative: Helbing, Jörg
(86) International application number: PCT/JP2005/022151
(87) International publication number: WO 2006/059703

(57) **Abstract**

The present invention is directed to provide a catalyst for reduction, which is capable of reducing even mono-substituted alkynes to alkenes and does not require the coexistence (combined use) of a toxic compound, and a method for reduction from alkynes to alkenes using the catalyst; and relates to a palladium-supported polyethyleneimine compound obtained by contacting a polyethyleneimine, a palladium compound and hydrogen gas in an oxygen-free state, a method for producing the above palladium-supported polyethyleneimine compound, a catalyst for reduction comprising the above palladium-supported polyethyleneimine compound, and a method for reducing from alkynes to alkenes **characterized by** contacting an alkyne and hydrogen in the presence of a palladium-supported polyethyleneimine compound, which is obtained by reacting a polyethyleneimine, a palladium compound and hydrogen gas in an oxygen-free state.

## Description

### Technical Field

The present invention relates to a palladium-supported polyethyleneimine compound which is capable of selectively reducing alkynes to alkenes, a method for production thereof, and a method for reducing alkynes using the compound.

### Background Art

Heretofore, in the methods for synthesizing alkenes from alkynes, there had been a problem that the reaction does not terminate at the stage of alkene but the alkene is reduced up to alkane, because the resultant alkene is susceptible to hydrogen reduction. Afterward, a method in which a catalyst referred to as Lindlar catalyst, which was palladium-supported calcium carbonate poisoned with lead tetraacetate, and quinoline were used, was developed, and the method had been commonly used when alkenes were synthesized from alkynes. However, the method had such problems that sometimes an alkene could not be synthesized from a mono-substituted alkyne (a terminal alkyne), and also that post-treatment of the catalyst after use was complex due to use of highly toxic lead. Thus, development of a catalyst alternative to the Lindlar catalyst, which does not have such problems as described above, is desired.

On the other hand, palladium used in the Lindlar catalyst is a metal element belonging to the 10th group, and has been widely used as a catalyst for reduction reactions, disproportionation reactions, and the like. In particular, palladium of 0 valence has been frequently used for catalytic reduction reactions. Palladium of 0 valence has, however, such problems that it is difficult to isolate in a stable state, in a complex using a hetero atom other than phosphine as a ligand, and that reducing activity thereof is deactivated even if it can be isolated. By subsequent study, it has been found that the palladium catalyst can be isolated as a homogeneous complex by mixing polyethyleneimine and palladium acetate, and further that the complex can be isolated without deactivating reducing activity by conducting the mixing reaction in argon atmosphere. However, the complex cannot reduce mono-substituted alkynes specifically to alkenes, and further study was needed.

Considering the circumstances described above, development of a palladium catalyst, which is capable of reducing even mono-substituted alkynes to alkenes and does not require the coexistence of a toxic compound in the experiment system, has been desired.

### Disclosure of the Invention

### Problem to be Solved by the Invention

Under the circumstances described above, the present invention is directed to providing a catalyst for reduction, which is capable of reducing even mono-substituted alkynes to alkenes and does not require the coexistence (combined use) of a toxic compound, and also a method for reducing alkynes to alkenes using the catalyst.

### Means for Solving the Problem

The inventors of the present invention have, after intensively studying a way to solve the above problem, found that a palladium-supported polyethyleneimine compound, which is obtained by contacting a polyethyleneimine, a palladium compound and hydrogen gas in an oxygen-free state, can selectively reduce alkynes to alkenes, and accomplished the present invention.

Namely, the present invention relates to "a palladium-supported polyethyleneimine compound obtained by contacting a polyethyleneimine, a palladium compound and hydrogen gas in an oxygen-free state", "a method for producing a palladium-supported polyethyleneimine compound, which comprises contacting a polyethyleneimine, a palladium compound and hydrogen gas in an oxygen-free state", "a catalyst for reducing from alkines to alkenes comprising a palladium-supported polyethyleneimine compound obtained by reacting a polyethyleneimine, a palladium compound and hydrogen gas in an oxygen-free state", and "a method for reducing from alkynes to alkenes characterized by contacting an alkyne and hydrogen in the presence of a palladium-supported polyethyleneimine compound obtained by reacting a polyethyleneimine, a palladium compound and hydrogen gas in an oxygen-free state".

### Effect of the Invention

The palladium-supported polyethyleneimine compound of the present invention is obtained by contacting a polyethyleneimine, a palladium compound and hydrogen gas in an oxygen-free state. The thus prepared palladium-supported polyethyleneimine compound is capable of selectively reducing alkynes to alkenes. In particular, although the reduction from mono-substituted alkynes to alkenes was difficult even with the Lindlar catalyst, which had been conventionally used in the reduction method, use of the palladium-supported polyethyleneimine compound of the present invention enables selective reduction to alkenes. Further, since the palladium-supported polyethyleneimine compound of the present invention can achieve the obj ect without using toxic lead in combination like the Lindlar catalyst, the compound has an effect that post-treatment after use is easy. In addition, the method for reducing from alkynes to alkenes of the present invention is a method using the above palladium-supported polyethyleneimine compound of the present invention as a reduction catalyst, and so the method has the same effect as described above.

### Best Mode for Carrying Out the Invention

As the polyethyleneimine of the present invention, any of a commercial product or those prepared by known method such as those obtained by ring-opening polymerization of ethyleneimine, those obtained by polycondensation of ethylene chloride and ethylenediamine, those obtained by thermal reaction of 2-oxazolidone, or the like, may be used. In addition, since raw material of these polyethyleneimines has an extremely high reactivity, the resultant polyethyleneimine (polymer) may be, for example, a linear structural unit or a branched structural unit having a mixture of primary, secondary and tertiary amino groups as shown by the following formulae: 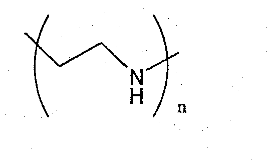 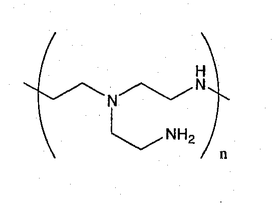 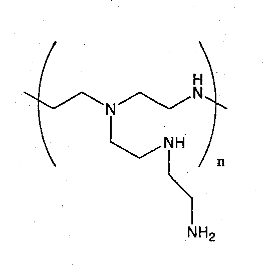 (wherein n represents a positive integer); and any ratio of these structural units or primary to tertiary amines may be acceptable.

Specifically, the polyethyleneimine includes, for example, a compound represented by the following general formula [1]: 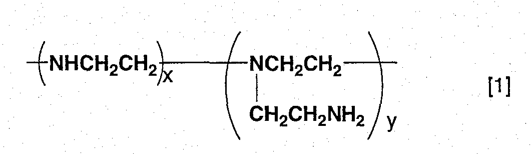 (wherein X and y each independently represents 0 or a positive integer). Molecular weight of the polyethyleneimine of the present invention is generally 100 to 10,000,000, preferably 800 to 750,000, more preferably 10,000 to 100,000, and further more preferably 20,000 to 30,000.

The palladium compound of the present invention includes, for example, palladium metal; for example, palladium oxides such as palladium dioxide and the like; for example, palladium halides such as palladium chloride, palladium bromide, palladium iodide, and the like; for example, ammonium palladates such as ammonium hexachloropalladate, ammonium tetrachloropalladate, and the like; for example, potassium halogenated palladates such as potassium hexachloropalladate, potassium tetrachloropalladate, potassium tetrabromopalladate, and the like; for example, sodium halogenated palladates such as sodium hexachloropalladate, sodium tetrachloropalladate, and the like; allyl palladium halide dimers such as bis (acetylacetonate) palladium, propyl palladium chloride dimer, and the like; 1,1-bis(diphenylphosphino) ferrocenedichloropalladium; bis(triphenylphosphine)palladium acetate; diamine dichloropalladium; trans-dichloro-bis(triphenylphosphine) palladium; palladium nitrate; palladium sulfate; palladium acetate; palladium complexes coordinated with ligand; and the like. Among them, preferable one is palladium chloride; allyl palladium halide dimers such as propylpalladium chloride dimer and the like; 1,1-bis(diphenylphosphino)ferrocene dichloropalladium; bis(triphenylphosphine)palladium acetate; diamine dichloropalladium; trans-dichloro-bis(triphenylphosphine) palladium; palladium nitrate; palladium acetate; potassium halogenated palladates; sodium halogenated palladates; and the like, and particularly preferable one is palladium acetate.

The above-described ligand of the palladium complexes coordinated with ligand includes, for example, 1,5-cyclooctadiene (COD), dibenzylideneacetone (DBA), norbornadiene (NBD), tricyclohexylphosphine (PCy₃), triethoxyphosphine (P(OEt)₃), tri-tert-butylphosphine (P(OtertBu)₃), tris-(o-tolyl)phosphine, bipyridine (BPY), phenanthroline (PHE), triphenylphosphine (PPh₃), 1,2-bis(diphenylphosphino)ethane (DPPE), triphenoxyphosphine (P(OPh)₃); trimethoxyphosphine (P(OCH₃)₃), bis(acetonitrile), bis (acetylacetonate), bis(benzonitrile), cycloocta-1,5-diene, ethylene (CH₂=CH₂), amine (NH₃), ethylenediamine (en) N₂, NO, PO₃ and the like. Among them, tris-(o-tolyl)phosphine, triphenylphosphine and the like are preferable.

The palladium-supported polyethyleneimine compound of the present invention is a compound, which is obtained by contacting a polyethyleneimine, a palladium compound and hydrogen gas in an oxygen-free state, and it is a black-colored syrupy substance. The term oxygen-free state here means a state in which air in the reaction system is replaced with an inert gas, hydrogen gas, mixed gas of both, or the like, or a state which is obtained by degassing using a vacuum pump commonly used in laboratory or the like (may be followed by replacing with an inert gas, hydrogen gas, mixed gas of both, or the like, if necessary). In other words, the term oxygen-free state means a state in which oxygen is removed in such a degree that the palladium-supported polyethyleneimine compound of the present invention can be prepared without any trouble, and does not necessarily mean an absolutely oxygen-free state. In addition, the above-described inert gas includes specifically, for example, helium, neon, argon, krypton, xenon, radon, nitrogen, and the like. Among them, argon is preferable. The polyethyleneimine to be used is preferably degassed in advance, to obtain further intensified oxygen-free state. Since polyethyleneimine usually contains air, this degassing of the air in polyethyleneimine is carried out.

Specific method to obtain the palladium-supported polyethyleneimine compound of the present invention will be described in the section of method for producing the palladium-supported polyethyleneimine compound below. The palladium-supported polyethyleneimine compound of the present invention obtained by such production method is a compound, which is effective for selective reduction of alkynes, in particular, mono-substituted alkynes to alkenes.

Production of the palladium-supported polyethyleneimine compound of the present invention is performed by contacting a polyethyleneimine, a palladium compound and hydrogen gas in an oxygen-free state, and may be preferably carried out as described below.

Namely, the above polyethyleneimine and the above palladium compound are brought into contact with each other in an oxygen-free state, and reacted generally at a temperature of 0 to 100ºC, preferably 10 to 40ºC, and under a pressure of generally 1 to 100 atm, preferably 1 to 10 atm, for generally 0.5 to 50 hours, preferably 5 to 40 hours in the presence of hydrogen gas, followed by drying to obtain the palladium-supported polyethyleneimine compound of the present invention.

In the above-described production method, reacting ratio of the polyethyleneimine and the palladium compound is not particularly limited so long as the palladium-supported polyethyleneimine compound of the present invention can be obtained at the ratio, but is generally 0.001 to 100 mol, preferably 0.1 to 50 mol, and more preferably 0.1 to 10 mol of palladium compound relative to one repeating unit (monomer unit) of polyethyleneimine.

In the above-described production method, contact of a polyethyleneimine and a palladium compound is preferably performed by contacting an organic solvent dissolving the polyethyleneimine with the palladium compound. The organic solvent dissolving a polyethyleneimine is preferably those in which a palladium metal is easily reduced, for example, alcohols and the like is preferable, and specifically, methanol, ethanol, propanol, isopropanol, tert-butanol, and the like are included. Among them, methanol and the like are preferable. In this case, amount of the organic solvent to be used is generally 1 to 100 mL, preferably 30 to 60 mL, more preferably 40 to 50 mL, and further more preferably 45 to 50 mL relative to 1 g of polyethyleneimine. In addition, as described above, the polyethyleneimine is preferably degassed before being dissolved into the organic solvent to intensify the oxygen-free state. The oxygen-free state in the production method may be any one of degassed state, under an inert gas atmosphere, under hydrogen gas atmosphere, or in the presence of both, as described above, but particularly preferable state is under an inert gas atmosphere. Namely, method for contacting and reacting a polyethyleneimine and a palladium compound in the above-described production method is preferably to contact a polyethyleneimine and a palladium compound under an inert gas atmosphere, and the inert gas is then replaced with hydrogen gas to promote the reaction. In addition, pressure of the above inert gas or hydrogen gas is generally 1 to 100 atm, preferably 1 to 10 atm, more preferably 1 to 3 atm, further more preferably 1 to 2 atm. Drying in the above production method is not particularly limited so long as the method is commonly used one in this field, but preferably conducted at a temperature of generally 10 to 40°C, preferably 15 to 25°C, under reduced pressure.

The production method of the palladium-supported polyethyleneimine compound of the present invention is explained more specifically as follows. Namely, an organic solvent dissolving a polyethyleneimine and a palladium compound are brought into contact under an inert gas atmosphere, then the inert gas is replaced with hydrogen gas, followed by reacting at a temperature of generally 10 to 40ºC, preferably 15 to 25ºC, for 10 to 40 hours, preferably 20 to 30 hours under hydrogen atmosphere, and the resultant reaction solution is dried under reduced pressure to obtain the palladium-supported polyethyleneimine compound of the present invention. In addition, palladium content in the thus obtained palladium-supported polyethyleneimine compound of the present invention varies depending on types of polyethyleneimine and palladium compound and ratio thereof, but generally 0.001 to 50% by weight, preferably 0.5 to 20% by weight, more preferably 1.0 to 10% by weight, and further more preferably 2.0 to 10% by weight of the total weight.

The catalyst for reducing from alkynes to alkenes of the present invention comprises the palladium-supported polyethyleneimine compound of the present invention. By using the catalyst, alkynes can be specifically reduced to alkenes.

The method for reducing from alkynes to alkenes of the present invention can be performed by contacting an alkyne and hydrogen in the presence of the palladium-supported polyethyleneimine compound of the present invention, and contact of an alkyne and hydrogen is preferably carried out in an organic solvent. Specifically, an alkyne and the palladium-supported polyethyleneimine compound of the present invention are added into an organic solvent, and the mixture is stirred and reacted at a temperature of generally 0 to 100ºC, preferably 10 to 40ºC, under a pressure of generally 1 to 10 atm, preferably 1 to 2 atm, for generally 0.5 to 50 hours, preferably 10 to 40 hours under hydrogen atmosphere. Thereafter, a solution containing an alkene is obtained from the resultant solution by solvent extraction, followed by drying the solution to obtain the desired alkene.

The alkynes to be used in the reduction method of the present invention may be any one of di-substituted alkynes (internal alkynes) and mono-substituted alkynes (terminal alkynes), and specific examples of di-substituted alkynes include but not limited to, for example,

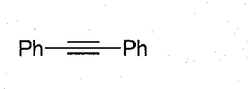

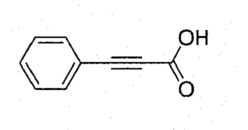

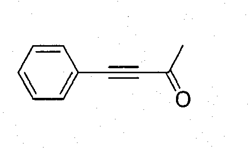

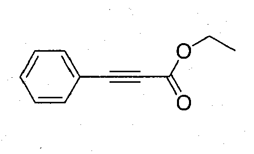

CH₃(CH₂)₄C≡C(CH₂)₄CH₃

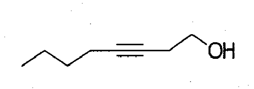

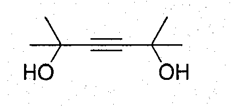

and the like, and specific examples of mono-substituted alkynes include but not limited to, for example,

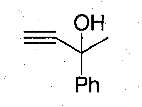

HC≡C(CH₂)₉CH₃

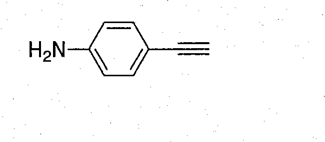

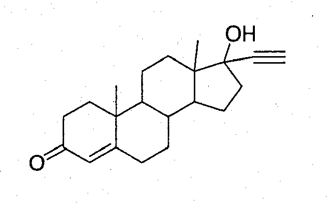

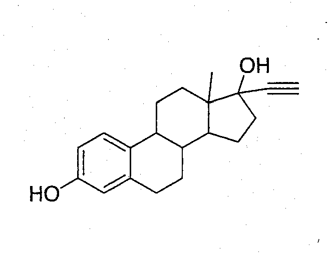

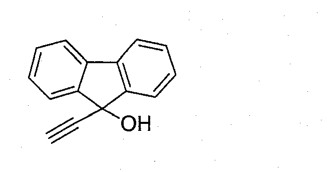

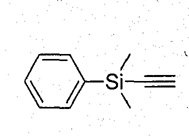

and the like.

In the reduction method of the present invention, amount of the palladium-supported polyethyleneimine compound of the present invention to be used is generally 0.1 to 50% by weight, preferably 5 to 20% by weight, and more preferably 10 to 15% by weight relative to the weight of an alkyne. Namely, palladium may be used in an amount of generally 0.0001 to 20% by weight, preferably 0.01 to 10% by weight, and more preferably 0.1 to 5% by weight relative to the weight of an alkyne.

In the reduction method of the present invention, the organic solvent, to which an alkyne and the palladium-supported polyethyleneimine compound of the present invention are added, is not particularly limited so long as the solvent can dissolve the alkyne and the palladium-supported polyethyleneimine compound of the present invention, and may be selected as appropriate so that higher production rate of alkene can be obtained depending on alkyne to be used. Specifically, the solvent include, for example, acetonitrile, ethyl acetate, dioxane, methanol, benzene, toluene, pyridine, tetrahydrofurane, hexane, diethyl ether, and the like. Among them, methanol, dioxane, ethyl acetate, pyridine, and the like are preferable due to versatility thereof, and dioxane is more preferable, and among dioxane, 1,4-dioxane is particularly preferable. In addition, the above organic solvent may be used in combination of two or more kinds. When two or more kinds of solvents are used in combination, preferable combination varies depending on type of alkene and the like, but includes combinations of ethyl acetate / dioxane, ethyl acetate / pyridine, methanol / dioxane, and the like, and among them, a combination of methanol / dioxane is preferable due to versatility thereof. When alkyne is an acidic compound, an aqueous solution of weak alkali such as potassium carbonate, calcium carbonate, and the like, or an organic base such as pyridine and the like is preferably added. When an aqueous solution of weak alkali is added, a water-soluble organic solvent such as methanol and acetone is preferably used as the above organic solvent. Further, when an aqueous solution of weak alkali is added, amount thereof to be added is preferably 1 to 3 equivalents to an alkyne, and when an organic base is added, amount thereof to be added is preferably 1 to 10 equivalents to an alkyne. In the reduction method of the present invention, amount of the above organic solvent to be used is generally 0.1 to 100 mL, preferably 1 to 50 mL, and more preferably 10 to 25 mL relative to 1 g of alkyne.

In the reduction method of the present invention, pressure of hydrogen gas to be used in the hydrogen atmosphere may be generally 1 to 3 atm, and preferably 1 to 2 atm.

In the reduction method of the present invention, solvent extraction may be performed according to the method known per se. Specifically, solvent extraction may be performed, for example, by adding, for example, ether or ester and water of generally 2 to 10 times in volume each of the resultant solution to the solution obtained after stirring, stirring the solution, leaving the solution at rest to separate into two layers, then taking out the organic solvent layer. In addition, ether to be used here includes, for example, diethyl ether, dipropyl ether, dibutyl ether, and the like, and ester to be used includes, for example, ethyl acetate, butyl acetate, propyl acetate, and the like. After the solvent extraction, the solution is more preferably washed using, for example, saturated saline of 1 to 2 times in volume of the solution containing the desired compound.

Method for drying in the reduction method of the present invention is not particularly limited so long as it is commonly used in this field, but drying is preferably performed at a temperature of generally 10 to 40ºC, and preferably 15 to 25ºC. In addition, prior to the drying, moisture is preferably removed using anhydrous magnesium sulfate or anhydrous sodium sulfate.

The reduction method of the present invention is described more specifically as follows. Namely, for example, an alkyne (1 g) and the palladium-supported polyethyleneimine compound (0.1 g) of the present invention are added to a mixed solvent of methanol (10 mL) and dioxane (10 mL), and the mixture is stirred for generally 10 to 40 hours, and preferably 20 to 30 hours, under hydrogen atmosphere. Thereafter, for example, 200 mL each of ethyl acetate and water are added to the resultant solution, which is then stirred and left at rest. The organic solvent layer is taken out. Saturated saline is added to the solution, and the organic solvent layer is taken out again. The resultant solution is dried by adding, for example, magnesium sulfate, filtered, and dried at room temperature under the reduced pressure, to obtain an alkene corresponding to the above alkyne.

### [Example]

### Example 1

### Preparation of syrupy palladium-supported polyethyleneimine (the Pd-PEI catalyst of the present invention)

A polyethyleneimine (2.11 g, produced by Sigma-Aldrich Japan K.K.) was poured into a 200 mL eggplant flask, and degassed under reduced pressure using a vacuum pump for 48 hours. Thereafter, methanol (100 mL, HPLC grade, produced by Wako Pure Chemical Industries Ltd.) was added to the eggplant flask to dissolve the polyethyleneimine homogeneously. The resultant solution was then transferred to another 200 mL eggplant flask, in which palladium acetate (200 mg, 1 mmol) had been added and then filled with argon in advance, and palladium acetate was completely dissolved in the polyethyleneimine / methanol solution. Subsequently, argon was replaced with hydrogen gas, and the solution was stirred at room temperature for 24 hours to promote the reaction. Methanol was then evaporated off under reduced pressure, and the residue was dried for 24 hours under reduced pressure using a vacuum pump, to obtain black-colored viscous solid (syrupy Pd-PEI catalyst of the present invention).

### Comparative Example 1

### Preparation of gum-like Pd-PEI catalyst

A polyethyleneimine (12.66 g, produced by Sigma-Aldrich Japan K.K.) was poured into a 200 mL Erlenmeyer flask, and methanol (60 mL) was added thereto to dissolve the polyethyleneimine. Palladium acetate (1.2 g, 6 mmol) was further added thereto. After argon replacement was carried out, the solution was stirred at room temperature for 26 hours. Thereafter, methanol was evaporated off under reduced pressure to obtain black-colored gum-like solid (gum-like Pd-PEI catalyst).

### Example 2

### Reduction of an alkyne using the syrupy Pd-PEI catalyst

3-Phenyl-1-butyn-3-ol (146 mg, 1 mmol) as a substrate and the syrupy Pd-PEI catalyst (15 mg) obtained in Example 1 were added to each of various solvents described in Table 1 (an amount described in Table 1), and subjected to a reaction by stirring the mixture at room temperature for 24 hours under hydrogen atmosphere. After completion of the reaction, ethyl acetate (20 mL) and water (20 mL) were added thereto and mixed. After leaving the mixture at rest, the ethyl acetate layer was taken out. After adding and mixing saturated saline (20 mL) to the resultant ethyl acetate layer, and leaving the mixture at rest, the ethyl acetate layer was taken out. Thereafter, the layer was dried with magnesium sulfate, and the solvent was evaporated off under reduced pressure.
The resultant substance was analyzed by ¹H-NMR spectra to determine residual ratio of the substrate, production rate of corresponding alkene and corresponding alkane. The obtained results are shown in Table 1. In addition, 1:2:3 in Table 1 represents a ratio of residual ratio of the substrate (3-phenyl-1-butyn-3-ol): production rate of corresponding alkene (3-phenyl-1-buten-3-ol): production rate of corresponding alkane (2-phenyl-2-butanol) (ratios by weight) .

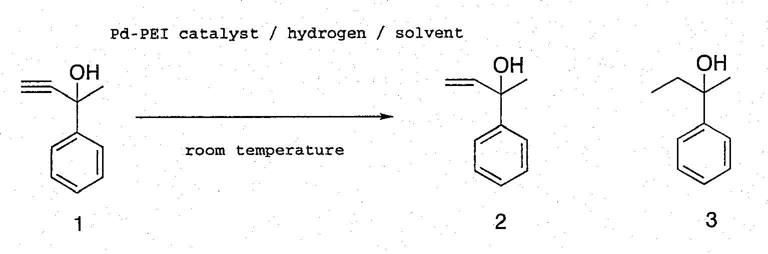

**[Table 1]**

| Solvent (Amount of Solvent) | 1:2:3 |
|---|---|
| Acetonitrile (1 mL) + 1,4-dioxane (1 mL) | 2 : 76 : 22 |
| Ethyl acetate (2 mL) + benzene (0.5 mL) | 4 : 82 : 14 |
| Ethyl acetate (2 mL) | 5 : 82 : 13 |
| Ethyl acetate (2 mL) + toluene (0.5 mL) | 0 : 86 : 14 |
| 1,4-Dioxane (2 mL) | 0 : 88 : 12 |
| Ethyl acetate (2 mL) + 1,4-dioxane (0.5 mL) | 0 : 91 : 9 |
| Ethyl acetate (2 mL) + pyridine (0.5 mL) | 0 : 96 : 4 |

From the results in Table 1, it is understood that high production rate of alkene is obtained in any solvent system, and that by using the catalyst of the present invention (the syrupy Pd-PEI catalyst), the alkene can be selectively formed from the alkyne. Among them, the case when ethyl acetate (2 mL) and pyridine (0.5 mL) were used as solvent can give particularly high production rate of the alkene and attain highly selective reduction.

### Comparative Example 2

### Reduction of an alkyne using the gum-like Pd-PEI catalyst

3-Phenyl-1-butyn-3-ol (146 mg, 1 mmol) as a substrate and the gum-like Pd-PEI catalyst (15 mg) obtained in Comparative Example 1 were added to a mixed solvent of water (1.25 mL) and acetonitrile (1.25 mL), and subjected to a reaction by stirring the mixture at room temperature for 48 hours under hydrogen atmosphere. After completion of the reaction, ethyl acetate (20 mL) and water (20 mL) were added thereto and mixed. After leaving the mixture at rest, the ethyl acetate layer was taken out. After adding and mixing saturated saline (20 mL) to the resultant ethyl acetate layer, and leaving the mixture at rest, the ethyl acetate layer was taken out. Thereafter, the layer was dried with magnesium sulfate, and the solvent was evaporated off under reduced pressure.
The obtained substance was analyzed by ¹H-NMR spectra to determine residual ratio of the substrate, production rate of corresponding alkene and corresponding alkane. The obtained results are shown in Table 2. Also, the results in Example 2 where ethyl acetate (2 mL) + pyridine (0.5 mL) were used as solvent are shown together. In addition, 1:2:3 in Table 1 represents a ratio of residual ratio of the substitute (3-phenyl-1-butyn-3-ol): production rate of corresponding alkene: production rate of corresponding alkane (ratios by weight) .

**[Table 2]**

| Catalyst | Substrate | Solvent | 1 : 2 : 3 |
|---|---|---|---|
| Example 2 (syrupy Pd-PEI catalyst) | 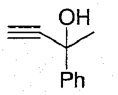 | Ethyl acetate (2 mL) + Pyridine (0.5 mL) | 0 : 96 : 4 |
| Comparative Example 2 (gum-like Pd-PEI catalyst) | 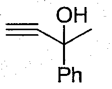 | Water (1.25 mL) + Acetonitrile (1.25 mL) | 0 : 0 : 100 |

From the results in Table 2, it is understood that a mono-substituted alkyne is reduced even up to alkane with the conventional gum-like Pd-PEI catalyst, whereas an alkyne can be reduced specifically to alkene by using the catalyst of the present invention (the syrupy Pd-PEI catalyst).

### Examples 3 to 9

### Reduction of various types of alkynes using the syrupy Pd-PEI catalyst

The same procedures as in Example 2 were carried out except that various types of alkynes described in Table 3 as a substrate and solvents (amounts of solvents) described in Table 3 instead of a mixed solvent of ethyl acetate (2 mL) and pyridine (0.5 mL) were used. The resultant substances were analyzed by ¹H-NMR spectra to determine residual ratios of the substrates, production rate of corresponding cis-type alkenes, trans-type alkenes and corresponding alkanes. The obtained results are shown in Table 3, respectively. In addition, 1:2:3:4 in Table 3 represents a ratio of residual ratio of the substrate: production rate of corresponding cis-type alkene: production rate of corresponding trans-type alkene: production rate of corresponding alkane (ratios by weight).

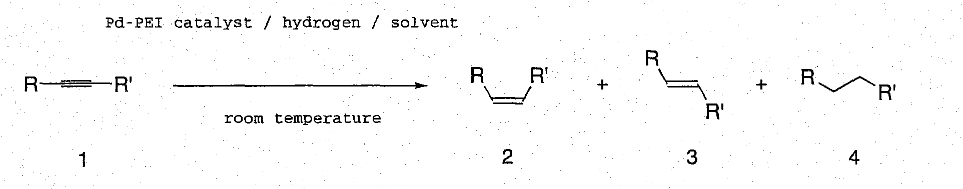

**[Table 3]**

| Example | Substrate | Kind of Solvent (Amount of Solvent) | 1 : 2 : 3 : 4 |
|---|---|---|---|
| 3 | 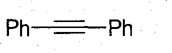 | Methanol (1 mL) + 1,4-Dioxane (1 mL) | 3 : 96 : trace : 1 |
| 4 | 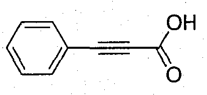 | Methanol (1 mL) + 1,4-Dioxane (1 mL) + Potassium Carbonate (1 eq.) | 0 : 100 : trace : trace |
| 5 | 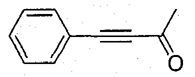 | Methanol (1 mL) + 1,4-Dioxane (1 mL) | 0 : 85 : 7 : 8 |
| 6 | 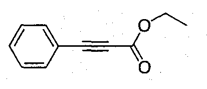 | Methanol (1 mL) + 1,4-Dioxane (1 mL) | 0 : 94 : trace : 6 |
| 7 | CH₃(CH₂)₄C≡C(CH₂)₄CH₃ | Methanol (1 mL) + 1,4-Dioxane (1 mL) | 0 : 100 : trace : 0 |
| 8 | 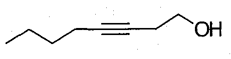 | Methanol (1 mL) + 1,4-Dioxane (1 mL) | 0 : 100 : 0 : 0 |
| 9 | 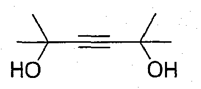 | Methanol (1 mL) + 1,4-Dioxane (1 mL) | 0 : 100 : trace : 0 |

From the results in Table 3, it is understood that any type of alkyne can be reduced specifically to alkene by using the catalyst of the present invention (the syrupy Pd-PEI catalyst), and also that reduction of di-substituted alkynes gives mostly cis-type alkenes.

### Examples 10 to 15

### Reduction of various types of mono-substituted alkynes using the syrupy Pd-PEI catalyst

The same procedures as in Example 2 were carried out except that various types of mono-substituted alkynes described in Table 4 as a substrate and solvents (amounts of solvents) described in Table 4 instead of a mixed solvent of ethyl acetate (2 mL) and pyridine (0 . 5 mL) were used. The resultant substances were analyzed by ¹H-NMR spectra to determine residual ratios of the substrates, production rates of corresponding alkenes and corresponding alkanes. The obtained results are shown in Table 4. In addition, 1:2:3 in Table 4 represents a ratio of residual ratio of substrate: production rate of corresponding alkene: production rate of corresponding alkane (ratios by weight).

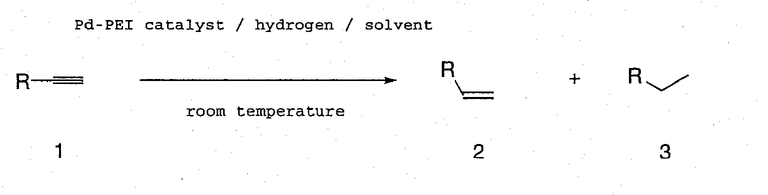

**[Table 4]**

| Example | Substrate | Solvent | 1 : 2 : 3 |
|---|---|---|---|
| 10 | HC≡C (CH₂)₉CH₃ | 1,4-Dioxane (2 mL) | 0 : 87 : 13 |
| 11 | 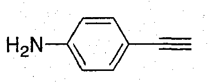 | 1,4-Dioxane (2 mL) | 11 : 85 : 4 |
| 12 | 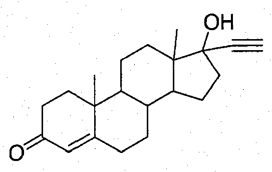 | Methanol (2 mL) + 1,4-Dioxane (0.5 mL) | 0 : 82 : 18 |
| 13 | 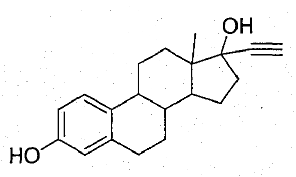 | Methanol (2 mL) + 1,4-Dioxane (0.5 mL) | 0 : 84 : 16 |
| 14 | 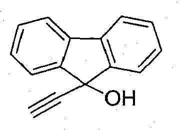 | Methanol (0.5 mL) + 1,4-Dioxane (2 mL) | 0 : 88 : 12 |
| 15 | 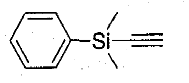 | Methanol (1 eq.) + 1,4-Dioxane (2 mL) | 1 : 77 : 22 |

### Comparative Examples 3 to 9

### Catalytic reduction of mono-substituted alkynes using the Lindlar catalyst

Each of various mono-substituted alkynes (1 mmol) described in Table 5 as a substrate and the Lindlar catalyst (palladium / potassium carbonate / lead tetraacetate, 10% by weight of each substrate) were added to a mixed solvent of cyclohexane (2 mL) and quinoline (117 µl), and the mixture was subjected to a reaction by stirring at room temperature for 24 hours under hydrogen atmosphere. The resultant reaction mixture was filtered with a membrane filter (Millex-LG, 0.20 µm, produced by Millipore Corp.), and ethyl acetate (20 mL), water (20 mL) and 10% sodium hydrogen sulfate (adequate amount) were added to the filtrate, followed by stirring. The solution was left at rest, and after separating into two layers, the ethyl acetate layer was taken out. Further, saturated saline (20 mL) was added to the resultant ethyl acetate layer, and stirred, and after separating into two layers,the ethyl acetate layer was taken out. The resultant ethyl acetate layer was dried with anhydrous magnesium sulfate, and the solvent was then evaporated off under reduced pressure.

The resultant substances were analyzed by ¹H-NMR spectra to determine residual ratios of the substrates, production rates of corresponding alkenes and corresponding alkanes. The obtained results are shown in Table 5. In addition, 1:2:3 in Table 5 represents a ratio of residual substrate: production rate of corresponding alkene : production rate of corresponding alkane (ratios by weight).

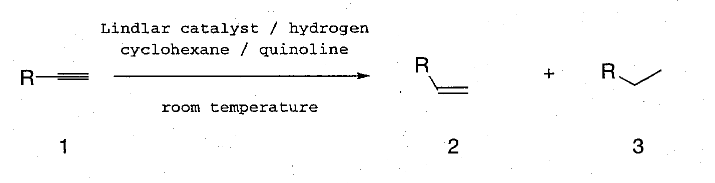

**[Table 5]**

| Comparative Example | Substrate | 1 : 2 : 3 |
|---|---|---|
| 3 | 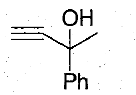 | 0 : 0 : 100 |
| 4 | HC≡C (CH₂) ₉CH₃ | 0 : 72 : 28 |
| 5 | 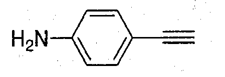 | 0 : 31 : 69 |
| 6 | 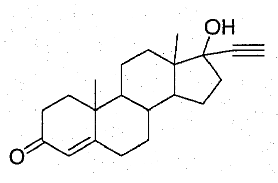 | 37 : 63 : trace |
| 7 | 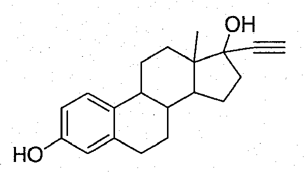 | 28 : 72 : trace |
| 8 | 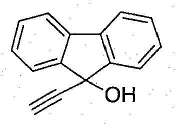 | 0 : 79 : 21 |
| 9 | 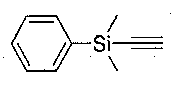 | 0 : 9 : 91 |

From the results in Table 5, the followings are understood. Namely, even with the Lindlar catalyst, which had been conventionally used for reduction from alkynes to alkenes, when alkene is synthesized from a mono-substituted alkyne, production rate of alkene becomes low, due to such problems that reduction to alkane occurs in high ratio and that substrate remains unreacted. On the other hand, as obvious from the results in Table 4, it is understood that alkene can be synthesized in a high production rate even from mono-substituted alkyne, by using the catalyst of the present invention (the syrupy Pd-PEI catalyst).

## Claims

1. A palladium-supported polyethyleneimine compound obtained by contacting a polyethyleneimine, a palladium compound and hydrogen gas in an oxygen-free state.

2. A method for producing a palladium-supported polyethyleneimine compound, which comprises contacting a polyethyleneimine, a palladium compound and hydrogen gas in an oxygen-free state.

3. The method according to claim 2, wherein the oxygen-free state is a state in which air in the reaction system is replaced with an inert gas, hydrogen gas or a mixed gas thereof, or a state obtained by degassing air in the reaction system.

4. The method according to claim 2, wherein molecular weight of the polyethyleneimine is 100 to 10,000,000.

5. The method according to claim 2, wherein the palladium compound is palladium acetate.

6. A catalyst for reducing from an alkyne to an alkene, comprising a palladium-supported polyethyleneimine compound, which is obtained by reacting a polyethyleneimine, a palladium compound and hydrogen gas in an oxygen-free state.

7. A method for reducing from an alkyne to an alkene, **characterized by** contacting an alkyne and hydrogen in the presence of a palladium-supported polyethyleneimine compound, which is obtained by reacting a polyethyleneimine, a palladium compound and hydrogen gas in an oxygen-free state.

8. The method for reduction according to claim 7, wherein an alkyne and hydrogen are contacted in an organic solvent in the presence of a palladium-supported polyethyleneimine compound.

9. The method for reduction according to claim 8, wherein the organic solvent is one kind of solvent or a mixture of two or more kinds of solvents selected from acetonitrile, ethyl acetate, dioxane, methanol, benzene, toluene, pyridine, tetrahydrofurane, hexane and diethyl ether.

10. The method for reduction according to claim 9, wherein the alkyne is a mono-substituted alkyne.
